# EUROPEAN PATENT APPLICATION

(11) **EP 4 145 132 A1**
(43) Date of publication of application: **08.03.2023**
(21) Application number: 21194798.1
(22) Date of filing: 03.09.2021
(51) Int. Cl.: G01N 33/569, G01N 33/68

(54) **METHODS AND KIT FOR DETERMINING THE ANTIBODY STATUS AND T-CELL IMMUNITY AGAINST SARS-COV-2**

(71) Applicant: ISAR Bioscience GmbH, 82152 Planegg (DE)
(72) Inventor: Ungerer, Martin, 82152 Planegg (DE); Holthoff, Hans-Peter, 82152 Planegg (DE); Tufts, Jennifer, 82152 Planegg (DE)
(74) Representative: Blodig, Wolfgang

(57) **Abstract**

The invention provides a kit and method for determining the antibody status, especially of neutralizing antibodies, against SARS-CoV-2 in a subject, said kit comprising:
(i) a solid support having attached an immobilized protein at least on a surface area of said solid support,
(ii) a soluble protein, and
(iii) a detection system for detecting binding of the soluble protein to the immobilized protein.

## Description

### FIELD OF THE INVENTION

The invention provides a kit for determining the antibody status, especially of neutralizing antibodies, against SARS-CoV-2 in a subject. The invention also provides a method for determining the antibody status, especially of neutralizing antibodies, against SARS-CoV-2 in a subject, such as a subject vaccinated against Covid-19. Moreover, the invention provides a method for determining the T-cell response and/or T-cell immunity to SARS-CoV-2 in a subject. The invention further provides a method of determining the immunization status against SARS-CoV-2 in a subject, comprising both methods of the invention.

### BACKGROUND OF THE INVENTION

Since December 2019, the SARS-CoV-2 pandemic has caused global health problems, leading to more than 4 million deaths (Johns Hopkins University database) and therefore demanding rapid development of vaccines for protection against the virus. Vaccine development has included mRNA, viral vectors, recombinant proteins and inactivated virus (Krammer, 2020), leading to discussions about the efficiency of the various approaches in terms of humoral and cellular immune responses against the virus. Several vaccines have been investigated in large clinical trials and have demonstrated safety and efficacy (Polack et al., 2020; Baden et al., 2020; Voysey et al., 2020): among them, BNT162b2 (Pfizer-BioNTech; mRNA), mRNA-1273 (Moderna; mRNA), and ChAdOx1 nCoV-19 (AZD1222) (Oxford-AstraZeneca; chimpanzee adenoviral vectored) have been approved globally and have been most frequently used in Europe. All of these vaccines have been designed to raise antibodies and T lymphocyte responses to the spike (S) protein, and all encode S sequences derived from the first reported SARS-CoV-2 sequence from Wuhan in January 2020 (Long et al., 2020).

Similar to other RNA viruses, SARS-CoV-2 is subject to progressive mutational changes. The pandemic spread leads to a huge extent of viral replication, increasing the odds that adaptive mutations will occur, which could lead to selective advantages, e.g. enhanced binding to human cells or immune escape from neutralizing antibodies (Volz et al., 2021). The S protein is a type-1 transmembrane glycoprotein, which may assemble into trimers (Walls et al., 2020). It is composed of two parts: the S1 domain bears the receptor-binding domain (RBD) and mediates cell binding via the angiotensin-converting enzyme-2 (ACE2) receptor, while the S2 domain completes membrane fusion, allowing the viral RNA to access the host cell cytoplasm to initiate viral replication.

The ACE2-RBD interaction is mediated by a small 25 amino acid patch of the RBD, which becomes accessible when the RBD moves into an upper direction (Hoffmann et al., 2020; Shang et al., 2020). Mutations in this region are most concerning: The alpha (B.1.1.7), beta (B.1.351), gamma (P.1) and delta (B.1.617.2) virus variants have been classified as variants of concern and have by far superseded the wt. These all have the potential to modulate ACE2-RBD binding affinity, potentially leading to an increased transmissibility. In addition, the variants' mutated amino acid residues can also modulate neutralization of SARS-CoV-2 by natural or vaccine-induced antibody responses.

Infections with SARS-CoV-2 frequently proceed without symptoms, whereby people may not be aware of a past SARS-CoV-2 infection and, accordingly, whether they have developed an immunization status that may protect them from future or further infections by (the same or another strain of) SARS-CoV-2. Further, the immunization status of vaccinated subjects generally decreases over time months after vaccination or may, particularly in the elderly or in immunosuppressed patients, not have developed sufficiently despite of vaccination. Therefore, the question arises for many subjects whether they are sufficiently immunized and whether a first or further vaccination should be considered. On the one hand, subjects sufficiently immunized should not be burdened by unnecessary further vaccinations. On the other hand, subjects not sufficiently immunized may wish to boost immunization by a further vaccine shot, notably if they belong to a patient group at risk for severe Covid-19.

It is therefore an object of the invention to provide a kit and method allowing quick and easy determination of the immunization status, such as antibody status or T-cell immunity. The kit and method should ideally allow automation so that the immunization status in a subject, notably in many subjects, can be determined routinely at low cost.

### SUMMARY OF THE INVENTION

These objects are accomplished by:
(1) A kit for determining the antibody status, especially of neutralizing antibodies, against SARS-CoV-2 in a subject, comprising:
   (i) a solid support having attached an immobilized protein at least on a surface area of said solid support,
   (ii) a soluble protein, and
   (iii) a detection system for detecting binding of the soluble protein to the immobilized protein;
   wherein
   (a) said immobilized protein is or comprises ACE2 or a fragment capable of binding to a receptor binding domain (RBD) of an S protein of SARS-CoV-2, and said soluble protein is or comprises an RBD of the S protein of SARS-CoV-2, or
   (b) said immobilized protein is or comprises an RBD of an S protein of SARS-CoV-2, and said soluble protein is or comprises ACE2 or a fragment thereof capable of binding to the RBD of the S protein of SARS-CoV-2.
(2) The kit according to item 1, wherein said soluble protein is biotinylated and the detection system comprises a streptavidin-labeled detection protein, such as a streptavidin-labeled peroxidase.
(3) The kit according to item 1 or 2, comprising, as said RBD, a first RBD of the S protein of a first SARS-CoV-2 strain and a second RBD of the S protein of a second SARS-CoV-2 strain, wherein said second RBD is a mutated variant of said first RBD, for determining the immunization or antibody status of the subject against the second SARS-CoV-2 strain compared to the immunization or antibody status of the subject against the first SARS-CoV-2 strain.
(4) The kit according to any one of items 1 to 3, wherein said solid support has attached:
   a first RBD of the S protein of a first SARS-CoV-2 strain on a first surface area of said support, and
   a second RBD of the S protein of a second SARS-CoV-2 strain on a second, separate, surface area of said support, and
   optionally RBDs of further SARS-CoV-2 strains on further separate surface areas of said support.
(5) The kit according to any one of items 1 to 4, wherein said solid support is a microarray having attached two or more spots of said immobilized protein.
(6) The kit according to item 4, wherein said solid support is a microarray having attached one or more spots of said first RBD and one or more spots of said second RBD, and optionally one or more further spots of further variants of said RBD.
(7) The kit according to any one of items 1 to 6, wherein said RBD is produced by a process comprising cultivating a mammalian cell containing a nucleic acid molecule comprising a polynucleotide encoding said RBD and expressing said RBD in said cell.
(8) The kit according to item 7, said RBD having a mammalian-type glycosylation.
(9) The kit according to any one of items 1 to 7, wherein said immobilized protein comprises a His-tag and said solid support is a Ni-NTA solid support.
(10) A method for determining the antibody status, especially of neutralizing antibodies, against SARS-CoV-2 in a subject, comprising:
   (A) providing a solid support having attached an immobilized protein,
   (B) mixing a blood or serum sample derived from said subject with a soluble protein,
   (C) contacting the mixture obtained in step (B) with a surface area of said solid support, said surface area having attached said immobilized protein, and
   (D) detecting binding of said soluble protein to said immobilized protein using a detection system;
   wherein
   (a) said immobilized protein is or comprises ACE2 or a fragment capable of binding to a receptor binding domain (RBD) of an S protein of SARS-CoV-2, and said soluble protein is or comprises an RBD of the S protein of SARS-CoV-2, or
   (b) said immobilized protein is or comprises an RBD of an S protein of SARS-CoV-2, and said soluble protein is or comprises ACE2 or a fragment thereof capable of binding to the RBD of the S protein of SARS-CoV-2.
(11) The method of item 10, wherein:
   step (B) comprises preparing two or more different dilutions of said blood or serum sample, and mixing each of said two or more different dilutions with said soluble protein,
   step (C) comprises contacting each of the two or more mixtures obtained in step (B) with a separate surface area of said solid support, each surface area having attached said immobilized protein, and
   step (D) comprises detecting binding of said soluble protein to said immobilized protein using a detection system on each surface area that was contacted with a mixture obtained in step (B).
(12) The method of items 10 or 11, wherein said soluble protein is biotinylated and the detection system comprises a streptavidin-labeled detection protein, such as a streptavidin-labeled peroxidase.
(13) The method according to any one of items 10 to 12, wherein said method is a microarray-immunoassay (MIA) using a flow-through chemiluminescence immunochip as said solid support.
(14) The method according to any one of items 10 to 13, wherein said subject is vaccinated against Covid-19, has recovered from Covid-19, or was previously infected by SARS-CoV-2.
(15) A method of determining the T-cell immunity or T-cell response to SARS-CoV-2 in a subject, comprising:
   (i) obtaining a blood sample from the subject,
   (ii) isolating peripheral blood mononuclear cells from said blood sample,
   (iii) incubating said cells obtained in step (ii) with a SARS-CoV-2 S protein, an RBD fragment of an S protein, or one or more peptides from an S protein or an RBD of SARS-CoV-2,
   (iv) staining extracellular T-cell markers selected from the group consisting of CD3, CD4, and CD8 of the cells obtained in step (iii),
   (v) fixing the cells obtained in step (iv) on a solid support,
   (vi) staining intracellular T-cell markers selected from the group consisting of IFN, TNF, and IL-2, followed by resuspending the cells, and
   (vii) analyzing extracellular and intracellular T-cell markers of resuspended cells by flow cytometry using signals due to the stained markers.
(16) The method according to item 15, wherein at least the extracellular marker CD4 and one of said intracellular markers are analyzed in step (vii).
(17) The method according to any one of items 15 or 16, wherein at least the extracellular markers CD3, CD4, and CD8 and one of said intracellular markers are analyzed in step (vii).
(18) The method according to any one of items 15 to 17, wherein said SARS-CoV-2 S protein, said RBD fragment of an S protein, or said one or more peptides from an S protein or an RBD are from the wild-type SARS-CoV-2 strain or from a mutant SARS-CoV-2 strain, such as the alpha, beta, gamma, or delta SARS-CoV-2 strain.
(19) The method according to any one of items 15 to 18, further comprising step (viii):
   comparing fluorescence intensities, analyzed in step (vii), of stained T-cell markers or the cell population stained for extracellular and intracellular T-cell marker(s) of said subject with a control.
(20) The method according to item 19, further comprising step (viii): comparing fluorescence intensities, analyzed in step (vii), of stained T-cell markers or the cell population stained for extracellular and intracellular T-cell marker(s) of said subject with the respective intensities or cell population of a vaccinated control subject or an average of recently vaccinated control subjects.
(21) The method according to item 19 or 20, comprising, in step (iii),
   incubating said cells with a SARS-CoV-2 S protein, an RBD fragment of an S protein, or one or more peptides from an S protein or an RBD of the wild-type SARS-CoV-2 strain in a first container and,
   incubating said cells with a SARS-CoV-2 S protein, an RBD fragment of an S protein, or one or more peptides from an S protein or an RBD of a mutant SARS-CoV-2 strain in a second container, and
   comprising in step (viii) comparing stained T-cell marker(s) in said cells incubated in said first container with that/those incubated in said second container.
(22) A method for determining the immunization status against SARS-CoV-2 in a subject, comprising the method according to item 10 and the method according to item 15, optionally as further defined in one or more dependent items.

The inventors have surprisingly found a fast and reliable way for determining the antibody status of a subject. In contrast to prior art methods that involve working with SARS-CoV-2 and thus require high safety measures, the method of the invention can be performed on a conventional ELISA plate format or automated on microfluid devices. In particular, the inventors have found that inhibition/neutralization assays using either the RBD of the SARS-CoV-2 S protein or ACE2 immobilized on a solid support, and the other in solution and mixed with sample, allow determining the antibody status of a subject. The results were comparable for the method of the invention. Both the direct interaction of anti-SARS-CoV-2 serum antibodies of vaccinated or naturally infected subjects with RBD, and the inhibition exerted by these antibodies on wt versus delta mutant RBD-ACE2 binding were measured by ELISA. It could not be expected that the impact exerted by immobilization of the immobilized protein, either ACE2 or the RBD, allows performing an inhibition assay, determining IC50 values (or other quantification), and reliable comparison with controls and/or RBD variants. The kit and method are such that the method can be formed using very small blood samples, such as 30 µl of finger-stick full blood. Results obtained were well comparable to those using sera obtained by venous puncture, showing that the assay can be upscaled to a rapid point-of-care ELISA.

The invention further provides a convenient method of determining T-cell immunity to SARS-CoV-2 in a subject. Also this method of the invention is simple and can be used using standard laboratory equipment, whereby it can be used routinely on T cells from blood samples from many subjects.

The findings of the inventors corroborate other recent studies which investigated delta mutant RBD by assays using live (pseudo)virus (Choi 2021, Liu 2021, Carreno 2021, Planas 2021, Kim 2021), and resulted in comparable ratios of delta mutant versus wild type binding. However, these assays are time-consuming and require specialized laboratory know-how. Although assessment of neutralization using in vitro binding assays may not reliably predict the potency of immune protection afforded by natural infection or immunization by real SARS-CoV-2, it can be carried out in non-specialized laboratories, and recent results using authentic virus largely substantiate the comparability (Skelly 2021). In over 90% of vaccinees, neutralizing titers (NT50) remained well above the level associated with immune protection in recent vaccine trials (Skelly 2021, Payne 2021). However, in many individuals post COVID-19 and recipients of a single dose of vaccine - neutralization capacity dropped. Reduced cross neutralization was observed against many virus variants and may impact on vaccine efficacy.

The inventors also investigated, using the method of the invention, T cell responses using peptide pools which were derived from the whole S sequence. This approach allowed for a broader investigation of anti-S protein immune responses. Generally, T cell responses to SARS-CoV2 are known to target a wide range of regions in the spike protein (Lumley 2020, Grifoni 2020). The inventors found that significant immune responses were elicited by all investigated vaccines including mRNA (Moderna and BioNtech), and the vector-based vaccination with Vaxzevria (AstraZeneca). The results show strong and comparable specific T cell responses to alpha (B1.1.7), beta (B1.351), gamma (P.1) and delta (B.1.617.2) spike protein variants in vaccinated subjects after two doses.

### BRIEF DESCRIPTION OF THE DRAWINGS

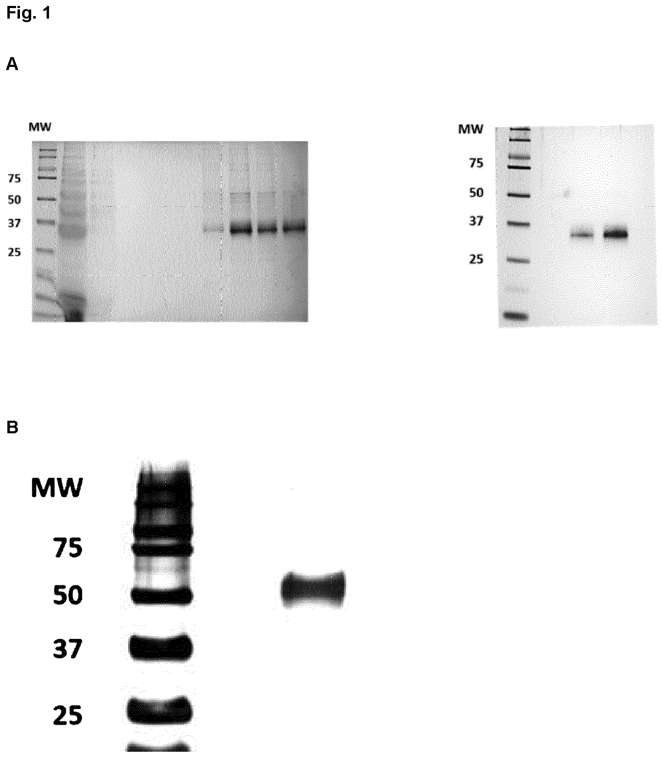

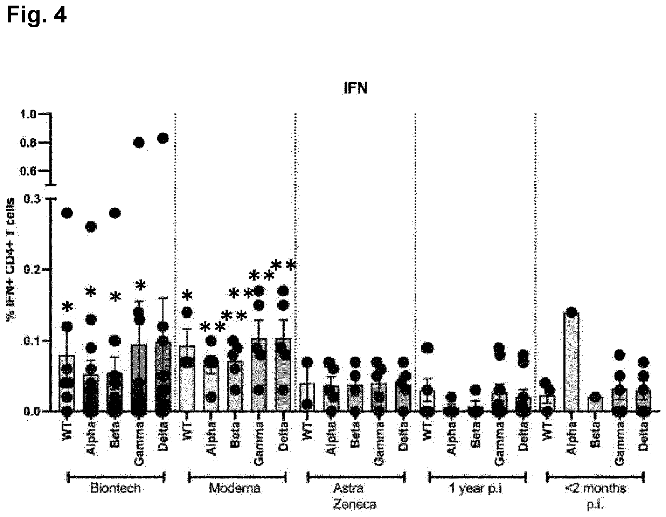

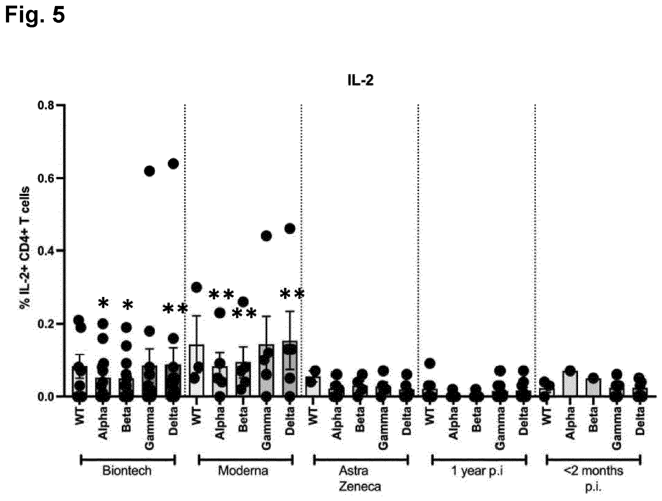

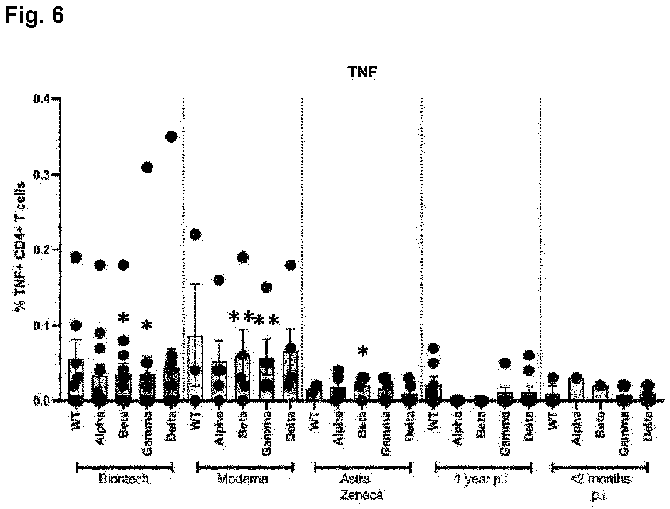

Fig. 1 Representative Coomassie gel electrophoresis (left panel) and silver stain (right panel) of SARS-CoV2 wild type spike RBD protein (A) and of nucleocapsid protein (B).

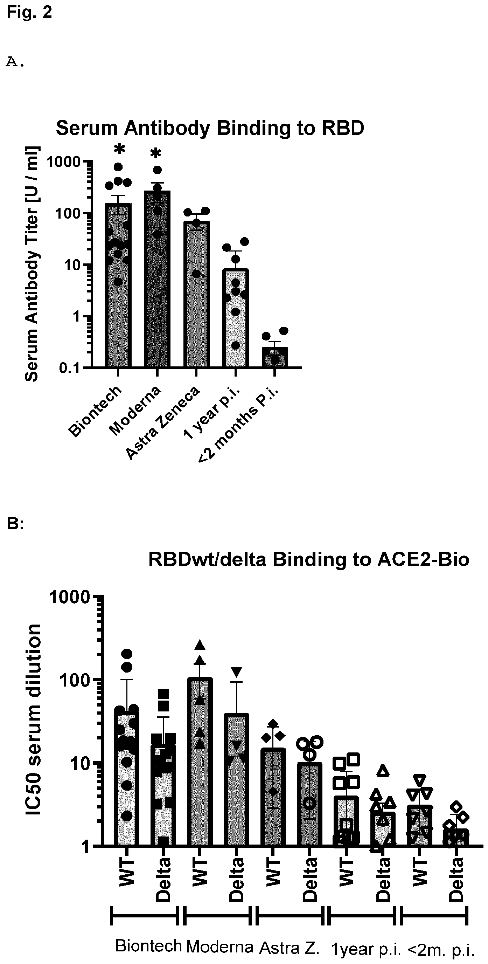

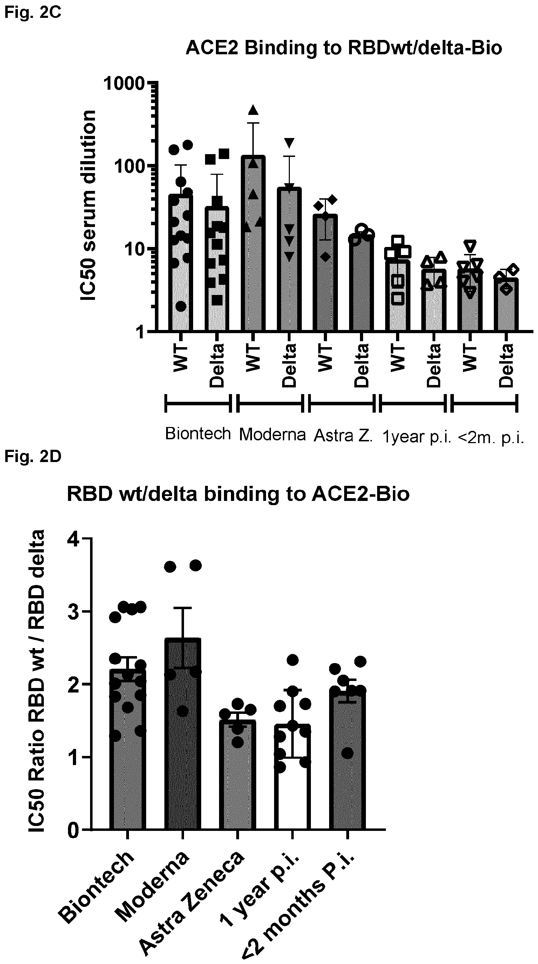

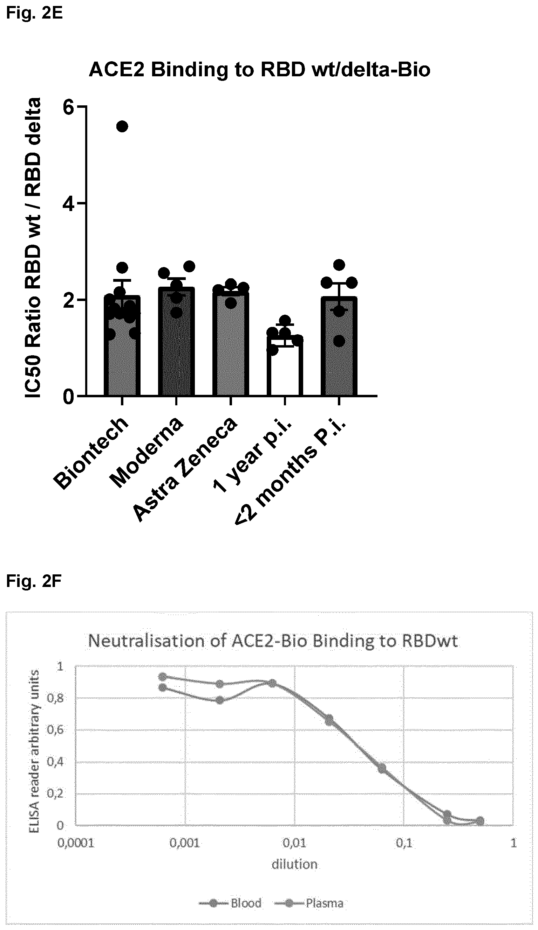

Fig. 2: The Figure shows results of n=13 independent individuals after vaccination with BioNtech (grey), n=5 Moderna (blue), n=5 AstraZeneca (AZ, red), and n=10 independent individuals one year (orange), and n=7 subjects < 2 months (green) after natural infection with standard errors of the mean (SEM). Significance was tested by one-way ANOVA (A) or by Kruskal-Wallis test for non-parametric values (B-E). **p < 0.05 vs. naturally infected subjects.
   A: Anti RBD and anti-nucleocapsid antibody titres; **B:** Absolute IC50-values calculated for the inhibition of biotinylated ACE2 binding to immobilized wt vs. delta mutant RBD. C: Absolute IC50-values calculated for the inhibition of biotinylated wt vs. delta mutant RBD binding to immobilized ACE2. D: Ratios between IC50-values calculated for inhibition of ACE2 binding to immobilized wt vs. delta mutant RBD. E: Ratios between IC50-values calculated for inhibition of wt vs. delta mutant RBD binding to immobilized ACE2. F: Read-out of a representative experiment showing the comparison between inhibition of biotinylated ACE2 binding to immobilized RBD with serum taken by venous sampling vs a drop of full blood.

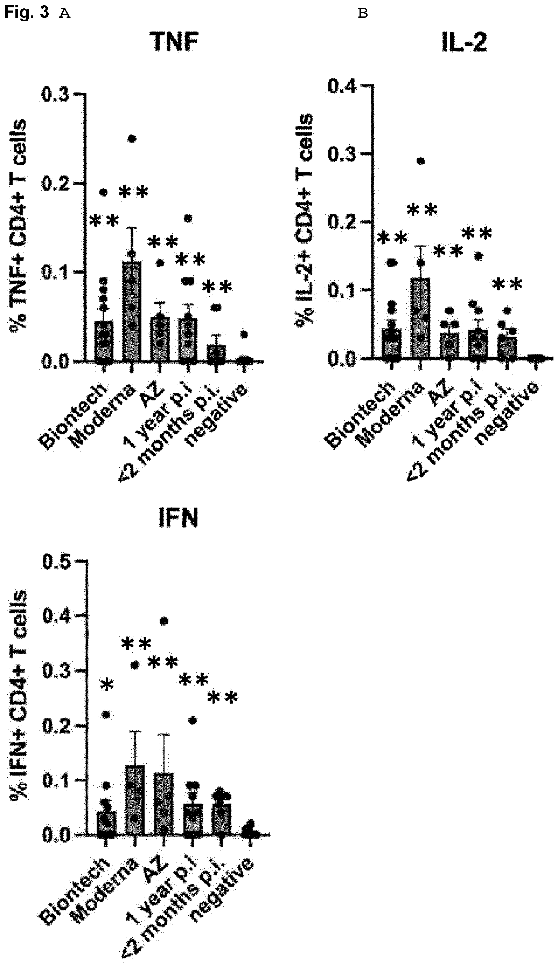

Fig. 3 Specific T cell responses elicited by the Miltenyi S protein peptide pool after vaccination with BioNtech (grey), Moderna (blue) or AstraZeneca (red), or one year (orange) or < 2 months (dark green) after naturally occurring infection with SARS-CoV2. In comparison, samples of healthy control subjects, who tested negative for SARS-CoV2-antibodies, are shown on the right of each panel. The Figure shows relative mean activation patterns of (A) tumour necrosis factor-alpha (TNF), (B) interleukin-2 (IL-2) and (C) interferon-gamma (IFN). The Figure shows results of n=13 independent individuals after vaccination with BioNtech, n=5 Moderna, n=5 AstraZeneca (AZ), and n=10 independent individuals one year and n=7 subjects < 2 months after natural infection with standard errors of the mean (SEM). For comparison, 27 negative healthy controls were included. Significance levels were tested by Kruskal-Wallis test for non-parametric values.
   **p < 0.01 vs. healthy controls
Fig. 4 Specific T cell responses elicited by the JPT peptide S protein pools after vaccination with BioNtech (grey), Moderna (blue) or AstraZeneca (red), or one year (orange) or < 2 months (dark green) after naturally occurring infection with SARS-CoV2. The Figure shows relative mean activation patterns of interferon-gamma (IFN) for wildtype, and for alpha (B.1.1.7), beta (B.1.351), gamma (P.1) and delta (B.1.617.2) mutants.
   The Figure shows results of n=13 independent individuals after vaccination with BioNtech, n=5 Moderna, n=5 AstraZeneca (AZ), and n=10 independent individuals one year and n=7 subjects < 2 months after natural infection with standard errors of the mean (SEM). Samples of non-infected individuals tested negative (not shown in the Figure to enhance legibility). Significance levels were tested with Kruskal-Wallis test for non-parametric samples. *p < 0.05 vs. healthy controls; **p < 0.01 vs. healthy controls and p < 0.05 vs 1 year after infection
**Fig. 5** Specific T cell responses elicited by the JPT peptide S protein pools after vaccination with BioNtech (grey), Moderna (blue) or AstraZeneca (red), or one year (orange) or < 2 months (dark green) after naturally occurring infection with SARS-CoV2. The Figure shows relative mean activation patterns of interleukin-2 (IL-2) for wildtype, and for alpha (B.1.1.7), beta (B.1.351), gamma (P.1) and delta (B.1.617.2) mutants.
   The Figure shows results of n=13 independent individuals after vaccination with BioNtech, n=5 Moderna, n=5 AstraZeneca (AZ), and n=10 independent individuals one year and n=7 < 2 months after natural infection with standard errors of the mean (SEM). Samples of non infected individuals tested negative (not shown in the Figure to enhance legibility). Significance levels were tested by Kruskal-Wallis test for non-parametric values. **p < 0.01 vs. healthy controls, and p < 0.05 vs. 1 year after infection
**Fig. 6** Specific T cell responses elicited by the JPT peptide S protein pools after vaccination with BioNtech (grey), Moderna (blue) or AstraZeneca (red), or one year (orange) or < 2 months (dark green) after naturally occurring infection with SARS-CoV2. The Figure shows relative mean activation patterns of tumour necrosis factor alpha (TNF) for wildtype, and for alpha (B.1.1.7), beta (B.1.351), gamma (P.1) and delta (B.1.617.2) mutants.
   The Figure shows results of n=13 independent individuals after vaccination with BioNtech, n=5 Moderna, n=5 AstraZeneca (AZ), and n=10 independent individuals one year and n=7 < 2 months after natural infection with standard errors of the mean (SEM). Samples of non-infected individuals tested negative (not shown in the figure to enhance legibility). Significance levels were tested by Kruskal-Wallis test for non-parametric values. *p < 0.05 vs. healthy controls, **p < 0.01 vs. healthy controls, and p < 0.05 vs. 1 year after infection

### DETAILED DESCRIPTION OF THE INVENTION

The method and kit for determining the antibody status of the invention make use of two proteins, which are the ACE2 protein or a fragment thereof and the RBD of the SARS-CoV-2 S protein. Among these two proteins, one is attached to the solid support and is referred to herein as immobilized protein. The other is used in solution and is referred to as soluble protein. Thus, there are two general embodiments of the method and kit. In a first general embodiment, the solid support has attached, as the immobilized protein, ACE2 or a fragment thereof, and said soluble protein is or comprises the RBD of the S protein of SARS-CoV-2. In the second general embodiment, the solid support has attached, as said immobilized protein, an RBD of an S protein of SARS-CoV-2, and said soluble protein is ACE2 or a fragment thereof. The second embodiment is preferred, as it is more suitable to perform multiple methods using two or more variants of the RBD in parallel using the same blood or serum sample (see further below) to determine the antibody status against two or more variants of SARS-CoV-2.

The kit for determining the antibody status requires a solid support to which the immobilized protein is attached. The solid support may be made of plastic or glass. In one embodiment, the solid support may be a multi-well plate such as a 96-well plate or a 384-well plate. Such plates are known in the art and are commercially available. There are multiple methods for attaching a protein to such multi-well plates, which are also known in the art. Preferably, the protein to be attached to the solid support has a His-tag and the solid support has on its surface groups such as Ni-NTA to which the His-tag can bind. For example, Ni-NTA Hissorb ELISA plates may be used that bind the protein to be immobilized via the His-tag to the plate.

The surface of the solid support may be divided into two or more surface areas, for example into 24 to 384 surface areas, each surface area preferably having attached an immobilized protein. Using multiple surface areas allows performing multiple methods of the invention is parallel. Generally, all surface areas of a given solid support have attached the same type of protein, i.e. all having attached either ACE2 or a fragment thereof, or all having attached an RBD of an S protein of SARS-CoV-2 or a protein comprising it. However, it is not necessary that all immobilized protein are the same. For example, where all surface areas have attached immobilized RBD, different RBDs may be attached to different surface areas, such as RBDs of different SARS-CoV-2 strains.

Where multiple surface areas have attached the same immobilized protein, two or more of the same reactions may be performed in parallel for increasing the accuracy of the result by averaging the individual determinations. In one embodiment, multiple blood or serum samples from different subjects may be determined in parallel on the same solid support. Alternatively or additionally, a solid carrier may have different RBDs attached to different areas for determining the antibody status against different SARS-CoV-2 strains of the subject (or of multiple subjects) in parallel.

The solid support having attached the immobilized protein may be a microarray having attached multiple spots of the protein in a predetermined order, whereby the multiple protein spots are spatially separated. Such solid support may be made of glass or plastic. The microarray may have at least 5, preferably at least 10, and more preferably at least 20 protein spots attached to it. For achieving high specificity for detecting binding of the soluble protein to the immobilized protein spots on the microarray, the immobilized protein(s) should be attached to the microarray in a small amount. Preferably, each protein spot on the microarray comprises 2 ng or less, preferably 1 ng or less, more preferably 0.5 ng or less protein.

The full-length ACE2 consists of an N-terminal peptidase domain (PD) and a C-terminal collectrin-like domain (CLD) that ends with a single transmembrane helix and a ~ 40-residue intracellular segment (doi.org/10.1101/2020.02.17.951848). For use in the invention, complete ACE2 or a fragment thereof may be used. The ACE2 or a fragment thereof must be capable of binding to a receptor binding domain (RBD) of an S protein of SARS-CoV-2. Whether this is the case may be tested experimentally, e.g. using a modification of the method of the invention, wherein no blood serum is used in step (B) for mixing with the soluble protein.

The ACE2 or a fragment thereof may comprise an N- or C-terminal tag or signal sequence that allows attaching to the solid support. C-terminal tags are preferred. As indicated above, the tag may be a His-tag (6xHis-tag) for allowing purification and/or binding to Ni-NTA-modified solid supports. Other tags or ways of modifying a protein for coating on solid surfaces are known in the art. Depending on the use of the ACE2 or its fragment in the invention, the ACE2 or a fragment thereof may be modified for allowing detection by the detection system. A preferred modification is biotinylation for allowing detection by a streptavidin-labeled detection protein. Biotinylation may be done as described in the Examples. In the Examples, a full-length ACE2 having a C-terminal His-tag was used, which is thus preferred. Such ACE2 is commercially available.

Thus, the term "ACE2" comprises proteins comprising the amino acid sequence of ACE2, and optionally additional amino acid sequences stretches at the N- or C-terminus and optionally further modifications. The ACE2 or its fragment is capable of binding to a receptor binding domain (RBD) of an S protein of SARS-CoV-2, such as the wt SARS-CoV-2 strain.

The RBD of the SARS-CoV-2 S protein is known in the art. A review on the SARS-CoV-2 S protein is found in Zhu et al. (doi.org/10.1002/jmv.27132). The RBD for use in the invention is a protein comprising a polypeptide whose amino acid sequence is or comprises:
(a) the amino acid sequence of SEQ ID NO: 1 *(wt RBD),* SEQ ID NO: 2 *(alpha RBD),* SEQ ID NO: 3 *(beta RBD),* SEQ ID NO: 4 *(gamma RBD),* or SEQ ID NO: 5 *(delta RBD);* or
(b) an amino acid sequence having at least 90%, preferably at least 95% sequence identity to the amino acid sequence of SEQ ID NO: 1, SEQ ID NO: 2, SEQ ID NO: 3, SEQ ID NO: 4, or SEQ ID NO: 5; or
(c) an amino acid sequence having at least 95%, preferably at least 97% sequence similarity to the amino acid sequence of SEQ ID NO: 1, SEQ ID NO: 2, SEQ ID NO: 3, SEQ ID NO: 4, or SEQ ID NO: 5); or
(d) an amino acid sequence having from 1 to 25, preferably from 1 to 12, amino acid substitutions, additions, deletions and/or insertions compared to the amino acid sequence of SEQ ID NO: 1, SEQ ID NO: 2, SEQ ID NO: 3, SEQ ID NO: 4, or SEQ ID NO: 5; or
(e) a truncated amino acid sequence of any one of those defined in items (a) to (d), said truncated amino acid sequence having a length of at least 80 %, preferably at least 90%, in terms of number of amino acid residues of any one of the amino acid sequence defined in (a) to (d) above. The truncated amino acid sequence preferably comprises the receptor binding motif of amino acids 437 to 508 of the S protein (cf. doi.org/10.1002/jmv.27132).

SEQ ID NO: 6 shows the amino acid sequence of the wt RBD with C-terminal His-tag. SEQ ID NO: 7 shows the amino acid sequence of the wt RBD with N-terminal signal sequence and C-terminal His-tag. The amino acid sequences of SEQ ID NOs: 6 and 7 are thus further alternatives for the definitions in above items (a) to (e). Analogously, the amino acid sequences of SEQ ID NOs: 2 to 5 may be complemented by a C-terminal His tag and/or the N-terminal signal peptide as shown in SEQ ID NO: 7.

Preferably, the RBD defined above, notably in (a), (b), (c), (d), and (e), is capable of binding to ACE2 or to an antibody produced in a mammal after infection with wt SARS-CoV-2 (Wuhan).

Irrespective of the RBD variants defined above in items (a), (b), (c), (d), and (e), the RBD defined with reference to SEQ ID NO: 2 preferably contains the N501Y mutation of the S protein. The RBD defined with reference to SEQ ID NO: 3 preferably contains the K417N, E484K and N501Y mutations of the S protein. The RBD defined with reference to SEQ ID NO: 4 preferably contains the K417T, E484K and N501Y mutations of the S protein. The RBD defined with reference to SEQ ID NO: 5 preferably contains the L452R and T478K mutations of the S protein.

The RBD may comprise an N- and/or C-terminal tag, signal sequence or other peptide as required. For binding to the solid support, the RBD may comprise a suitable tag, such as a His-tag (meaning a 6 x His-tag). The His-tag is preferably at the C-terminal end of the RBD. Alternatively or additionally, the RBD may have an additional sequence stretch at the N-terminal end, such as a signal sequence useful for expressing the RBD. Similarly as described above for ACE2, if the RBD is used as the soluble protein, the RBD may be modified for allowing detection by the detection system. A preferred modification is biotinylation for allowing detection by a streptavidin-labeled detection protein.

Where a protein is defined herein by a number or number range of amino acid substitutions, additions, deletions, and/or insertions, amino acid substitutions, additions, insertions or deletions may be combined, but the given number or number range refers to the sum of all amino acid substitutions, additions, insertions and deletions. Among amino acid substitutions, additions, insertions and deletions, amino acid substitutions, additions, and deletions are preferred. The term "insertion" relates to insertions within the amino acid sequence of a reference sequence, i.e. excluding additions at the C- or N-terminal end. The term additions means additions at the C- or N-terminal end of the amino acid sequence of a reference sequence. A deletion may be a deletion of a terminal or an internal amino acid residue of a reference sequence. Reference sequences are amino acid sequences identified herein by a SEQ ID NO.

Herein, the determination of sequence identities and similarities is done using Align Sequences Protein BLAST (BLASTP 2.6.1+) (Stephen F. Altschul, Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402).

The RBD may be expressed according to methods generally known in the art. A process of producing the RBD may comprise cultivating a mammalian cell containing a nucleic acid molecule comprising a polynucleotide encoding the RBD and expressing the RBD in said cell. The mammalian cell may be a human cell or an animal cell. Widely used mammalian cells for protein expression are CHO cells that are also used in the examples. Suitable culture media for culturing mammalian cells are known in the art. Methods of expressing a protein in mammalian cells are also known in the art. The polynucleotide encoding said RBD may be codon-optimized for expression in the cells used for producing the protein. The nucleic acid molecule may further contain genetic elements for expressing the protein from the polynucleotide, such as a promoter and/or a terminator. The nucleic acid molecule may be a vector or plasmid and may further contain a selectable marker. Use of a mammalian cell has the advantage that a mammalian-type glycosylation may be produced on the RBD. Thus, the RBD preferably comprises, in addition to the polypeptide of the RBD (and an optional tag and/or signal peptide) glycosylation, more preferably mammalian-type glycosylation. The RBD may be purified using generally known methods, e.g. using affinity chromatography making use of a His-tag of the RBD. After or during purification, the protein is transferred in a suitable buffer to be used as the soluble protein or in a buffer suitable for immobilization on the solid support. A solution of the soluble protein may be frozen or dried, e.g. by lyophilization for later use.

The kit comprises the soluble protein. The soluble protein may be contained in the kit in a dry form, e.g. lyophilized form, or in a solution. The solution is preferably an aqueous solution that may further contain a buffer. The solution may be present as a high concentration stock solution to be diluted for use in the method. In this case, the concentration of the solution may be from 0.01 to 10 mg/ml, preferably from 0.1 to 5 mg/ml. Alternatively, the kit may contain the solution with a concentration of the soluble protein ready for use in the method. In this case, the concentration may from 0.1 to 20 µg/ml, preferably from 0.2 to 10 µg/ml, more preferably from 0.4 to 2 µg/ml.

For producing the solid support having attached the immobilized protein, the protein to be immobilized is attached to surface areas of the solid support. The surface chemistry for attaching a protein on a solid support, e.g. to form a microarray, is known in the art. These aspects are described in detail e.g. in the dissertation of Klaus Wutz at the Technical University Munich, 2014. Parts of the dissertation were published by Wutz et al., Analytical Chemistry 2013, 85, 5279-5285. Particularly preferred is the surface chemistry used and described in the latter publication (see also supporting information therein).

Detection systems for detecting binding of the soluble protein to the immobilized protein are generally known in the art. A detection system may comprise, as a detection protein, an enzyme that can bind to the soluble protein and that can produce a color or light signal, optionally from a suitable substrate of the enzyme. For the invention, a peroxidase may be used as a detection protein. The detection protein may comprise a tag allowing binding of it to the soluble protein. The tag may be streptavidin that allows binding of the detection protein to a biotinylated soluble protein. Thus, a preferred detection system comprises a streptavidin-labeled peroxidase. As generally known, peroxidase can oxidize substrates by peroxide, whereby a colored or luminescent product is produced. The color or luminescence produced is thus related to the binding of the detection protein to the soluble protein and, in turn, related to the binding of the soluble protein to the immobilized protein. Developing color or luminescence may be measured by a suitable camera, and color intensity or luminescence intensity may be read out using a plate reader.

The method for determining the antibody status comprises at least steps (A) to (D). In step (A), the solid support having attached an immobilized protein is provided. The solid support, attaching of the immobilized protein to it, and the proteins that may be immobilized was described above. After attaching, the immobilized protein, the coated solid support is preferably washed, e.g. with PBST (PBS, 0.1 % Tween-20) and then blocked with a blocking solution (PBST, 3% milk powder).

In step (B), a blood or serum sample derived from a subject is mixed with the soluble protein. For this purpose, the soluble protein is generally present in solution, preferably in aqueous solution. A blood sample is obtained from the subject the antibody status of which is to be determined. The subject is generally a human subject, although the method is not limited to human subjects and may as well be carried out with mammalian subjects in general. The subject may be vaccinated against Covid-19, may have recovered from Covid-19, or may previously have been infected by SARS-CoV-2. The blood sample may be full blood, e.g. venous blood. The blood is preferably taken from the finger-tip of a patient. For the method (also referred to herein as "assay"), a small volume of blood is sufficient. The amount of blood needed for the method is in the range of 5 to 100 µl, preferably 10 to 20 µl. The assay may as well be carried out with serum prepared from the blood sample.

The mixing of step (B) may be done in one or more mixing ratios of the blood or serum sample with the soluble protein. Thus, the method preferably comprises preparing two or more different dilutions of said blood or serum sample with water or a suitable aqueous buffer (e.g. PBST), and mixing each of said two or more different dilutions with a given solution of the soluble protein, such as a given volume of the solution of the soluble protein. The blood or serum sample obtained from the subject may be diluted with water or a suitable aqueous buffer in a dilution series of, for example, 1:2, 1:4, 1:8, 1:16, 1:48, 1:160, 1:1480, 1:1600 by volume, and each dilution may be mixed with a fixed volume of the solution of the soluble protein. The concentration of the soluble protein in the resulting mixture may be from 0.05 to 10 µg/ml, preferably from 0.1 to 5 µg/ml, more preferably from 0.2 to 1 µg/ml.

In step (C), the mixture obtained in step (B) is contacted (incubated) with a surface area of said solid support, said surface area having attached an immobilized protein. Where two or more mixtures are prepared in step (B) with two or more dilutions of blood or serum sample, each mixture is contacted (incubated) with a different surface area having attached the immobilized protein. The contacting is done for a sufficient period of time for allowing binding of the soluble protein to the immobilized protein. The period of time should be at least 5 minutes, preferably at least 10, more preferably at least 30 minutes. The contacting may be done for about 1 hour. After expiry of the predetermined contacting or incubation time, the mixture is removed from the solid support and the support washed multiple times or rinsed to ensure complete or nearly complete removal of components of the mixture that have not bound to the immobilized protein on the support.

In step (D), binding of said soluble protein to said immobilized protein is detected using a detection system. Possible embodiments of the detection system were described above. Preferably, the detection system comprises a detection protein (e.g. peroxidase) that can bind to the soluble protein. The soluble protein may be biotinylated and the detection system may comprise a streptavidin-labeled detection protein, such as a streptavidin-labeled peroxidase. The detection protein in a suitable solvent is incubated with the solid support obtained in step (C) for allowing binding of the detection protein to soluble protein that is bound to the immobilized protein. After washing away unbound detection protein, a solution of the substrate of the detection protein, and any necessary co-substrate, is incubated for a predetermined period of time. Color or luminescence developed by the action of the detection protein on the substrate may then be read out using known methods, e.g. using a plate reader. Blank readings measured in negative control surface areas may be subtracted from the readings of surface areas where the determination of the assay is done.

The signal determined for a given surface area is a measure of the neutralization, by antibodies against the RBD in the blood or serum sample of the subject, of the binding of the RBD to ACE2. Thus, the signal is a measure for the antibody status against SARS-CoV-2 in the subject. The signals may be compared with signals with positive and/or negative controls, e.g. in positive and/or negative surface areas of the solid support. A positive control may be a sample form a subject known to have been recently vaccinated against Covid-19 or a sample containing a known amount of anti-SARS-Cov-2 antibodies. A negative control may be a sample from a subject known not to be vaccinated against Covid-19 and known not to have been infected by SARS-Cov-2.

As mentioned above, in a preferred embodiment, step (B) comprises preparing two or more different dilutions of said blood or serum sample, and mixing each of said two or more different dilutions with said soluble protein, step (C) comprises contacting each of the two or more mixtures obtained in step (B) with a separate surface area of said solid support, each surface area having attached said immobilized protein, and step (D) comprises detecting binding of said soluble protein to said immobilized protein using a detection system on each surface area that was contacted with a mixture obtained in step (B). In this case, from the various signals obtained in step (D), IC50 blood or serum dilution values may be determined that represent neutralizing serum dilutions. Such IC50 values may be compared with negative and/or positive control for determining the antibody status, against SARS-CoV-2 in the subject.

The assay of the invention may be done using two or more different RBDs in parallel on the same or on different solid supports. From the signals obtained in step (D), the antibody status of a subject against different strains of SARS-CoV-2 may be determined and/or compared. In this embodiment, it is preferred that the soluble protein is ACE2 or a fragment thereof and the two or more different RBDs are immobilized on different surface areas of the solid carrier. In this way, the mixing and contacting of steps (B) and (C) can be the same for all RBDs for a sample of a given subject, while a prepared solid carrier carrying the two or more different RBDs can be used. The method may be a microarray-immunoassay (MIA) using a flow-through chemiluminescence immunochip as said solid support. The immunochip may carry many different RBDs, optionally each on multiple surface areas. In this way, many assays may be performed in parallel, including assays using blood or serum samples from two or more different subjects.

In a preferred embodiment, the method is for determining the antibody status of neutralizing antibodies against SARS-CoV-2 in a subject. Neutralizing antibodies are antibodies that inhibit binding of the SARS-CoV-2 to ACE2 by binding to epitopes of the S protein that interact with ACE2. Since the RBDs used in the invention include the receptor binding motif (residues 437-508 of the S-protein), the assay of the invention predominantly tests for neutralizing antibodies and therefore preferably determines the antibody status of neutralizing antibodies against SARS-CoV-2 in a subject.

Based on the results of the method of the invention for a subject, it may be decided whether a first or further immunization by vaccination is necessary and/or useful for the subject. Thus, if the antibody status in a subject is found to be good or high, use of the method and kit allow avoiding unnecessary (further) vaccination of a subject. If the antibody status in a subject is found to be insufficient, the method and kit allow avoiding or ending the state of insufficient immunization by administering a first or further vaccination to the subject. Since the method may be performed in an automated manner with many assays in parallel, the kit and method can be used routinely on blood or serum samples of many subjects.

### Method of determining the T-cell response or T-cell immunity to SARS-CoV-2 in a subject

The invention provides a method of determining T-cell immunity to SARS-CoV-2 in a subject. The T-cell immunity may, more specifically, be determined by determining the T-cell response to SARS-CoV-2 in a subject.

In step (i) of the method, a blood sample is obtained from the subject. The subject is generally a human subject, although the method is not limited to human subjects and may as well be carried out with mammalian subjects in general. The subject may be vaccinated against Covid-19, may have recovered from Covid-19, or may previously have been infected by SARS-CoV-2. The blood sample is preferably from peripheral blood. The amount of blood needed for the method is in the range of from 1 to 10 mL, preferably 1 to 5 mL per subject.

In step (ii), peripheral blood mononuclear cells (PBMCs) are isolated from said blood sample, using a generally known method, such as using density gradient centrifugation. The PBMCs may be stained and counted, and be frozen in an aqueous solution, such FBS that may contain DMSO. PBMCs may be stored in liquid nitrogen.

In step (iii), the PBMCs obtained in item (ii) are incubated for stimulating the cells. There are many possibilities for stimulating the cells. In any event, the cells are incubated with an S protein of SARS-CoV-2, or a fragment thereof. A fragment of the S protein may be or comprise the RBD of the S protein. The RBD may be as defined above with reference to SEQ ID NOs: 1 to 5, optionally including or excluding added tags or signal peptides. A further possibility of a fragment is the use of one more peptides from the S protein. The term "peptide from the S protein" means that the peptide has an amino acid sequence also present in an S protein, preferably an RBD. A peptide for this embodiment may have a length of from 10 to 50, preferably from 10 to 20 amino acid residues. Preferably, multiple peptides, i.e. a peptide mixture, are used in combination for stimulating the cells. Multiple peptides may represent multiple regions of an S protein or an RBD along the primary sequence of an S protein or RBD. The number of peptides present in the mixture may be from 2 to 300, preferably from 5 to 200, more preferably from 10 to 150. Thus, in a preferred embodiment of step (iii), said cells are incubated with a peptide mixture from 5 to 200 peptides from an S protein or an RBD of SARS-CoV-2.

The PBMCs may be incubated and stimulated in step (iii) with an S protein or fragment(s) thereof from one SARS-CoV-2 strain, or with a mixture of S proteins or fragment(s) thereof from two or more different SARS-CoV-2 strains. Generally, it is preferred to incubate the cells with S protein or fragment(s) thereof from one SARS-CoV-2 strain. Optionally, two or more of the assays may be performed, wherein PBMCs may separately be incubated with different S proteins or fragments thereof. In this way, T-cell immunity of a subject may be determined against two or more SARS-CoV-2 strains, e.g. in parallel. The SARS-CoV-2 S protein, the RBD fragment of an S protein, or the one or more peptides from an S protein or an RBD are from the wild type SARS-CoV-2 strain or from a mutant SARS-CoV-2 strain, such as the alpha, beta, gamma, or delta SARS-CoV-2 strain.

In a preferred embodiment, step (iii) comprises:
incubating said cells with a SARS-CoV-2 S protein, an RBD fragment of an S protein, or one or more peptides from an S protein or an RBD of the wild-type SARS-CoV-2 strain in a first container and,
incubating said cells with a SARS-CoV-2 S protein, an RBD fragment of an S protein, or one or more peptides from an S protein or an RBD of a mutant SARS-CoV-2 strain in a second container, and
comprising in step (viii) comparing stained T-cell marker(s) in said cells incubated in said first container with that/those incubated in said second container.

Subsequently, the extracellular T-cell markers and intracellular T-cell markers of the stimulated cells are stained and the cells are then analyzed for these T-cell markers. Cells are stained for analysis by flow cytometry. For this purpose, the T-cell markers are stained with fluorescent dye-labeled antibodies, as commonly done in flow cytometry. The fluorescent dye-labeled antibodies against the markers to be analyzed in the invention are commercially available, cf. Table 1. The skilled person understands that different fluorescent dyes should be used for different markers to allow analysis for the different markers separately. However, in a case a joint signal for all markers or for a selected sub-group of markers is to be determined, it is possible to use the same fluorescent dye for two or more markers.

In step (iv), the cells are stained for the extracellular T-cell markers selected from the group consisting of CD3, CD4, and CD8 of the cells obtained in step (iii). Preferably, the cells are stained at least for the marker CD4, and optionally also for CD3 and/or CD8. The staining of these markers can be done as is known in the art. As indicated above, if more than one marker is stained, different fluorescent dyes should be used for allowing distinguishing the markers in step (vii).

In the subsequent step (v), the cells obtained in step (iv) are fixed on a solid support for staining intracellular T-cell markers in step (vi). The solid support may be made of glass or plastic, e.g. a multi-well plate. Fixing of cells on a solid support is done according to generally known methods. After fixing, cells should be washed.

In step (vi), intracellular T-cell markers selected from the group consisting of IFN, TNF, and IL-2 are stained. For this purpose, the fixed cells are preferably treated with a permeabilization buffer. The intracellular markers are then stained, e.g. using antibodies against these markers labeled with dyes shown in Table 1. Stained cells are then resuspended for subsequent step (vii).

The method of the invention has the great advantage that analyzing the cells having the stained T-cell markers can be done by flow cytometry. Commercial instruments can be used for this purpose. Flow cytometry allows analyzing individual cells in a sample for fluorescence at multiple wavelengths at the same time. Thus, the fraction of cells in the cell population of a sample obtained in step (vi) showing fluorescence for each of the T-cell markers to be investigated can be counted and determined, and be represented in dot plots or other diagrams. For example, the percentage of cells positive for at least one intracellular and at least one extracellular T-cell marker may be represented (e.g. as done in Figs. 2 to 6).

For determining the T-cell immunity, notably the T-cell response, in/of the subject, the fluorescence intensities, analyzed in step (vii), of stained T-cell markers or the cell population fraction stained for at least one extracellular and at least one intracellular T-cell marker may be compared with a positive control and/or a negative control. A positive control may be a sample from a subject known to have been recently vaccinated against Covid-19. A negative control may be a sample from a subject known not to be vaccinated against Covid-19 and known not to have been infected by SARS-Cov-2. Quantitative thresholds for the fraction of cells analyzed in step (vii) may be defined for considering a subject to have a good T-cell immunity or T-cell response.

Based on the results of the method of the invention for a subject, it may be decided whether a first or further immunization by vaccination is necessary and/or useful for the subject. Thus, if the T-cell immunity or T-cell response in a subject is found to be good or high, the method allows avoiding unnecessary (further) vaccination of a subject. If the antibody status in a subject is found to be low or insufficient, the method allows avoiding or ending the state of insufficient immunization by administering a first or further vaccination to the subject. Since the method may be performed using standard laboratory equipment, the method can be used routinely on blood samples of many subjects.

The method for determining the antibody status, especially of neutralizing antibodies, against SARS-CoV-2 in a subject and the method for determining the T-cell response to SARS-CoV-2 in a subject may be combined and both be performed on the same subject for obtaining improved information on the immunization status of the subject against SARS-CoV-2.

### EXAMPLES

### Research Subjects

Blood was collected from 67 volunteers with informed consent, who had been previously infected with SARS-CoV-2 corona virus or been vaccinated against the virus or been unexposed. SARS-CoV-2 infection was confirmed by PCR testing. Vaccination had been completed with two full doses in all vaccinees, and blood was sampled 5.2+/- 0.9 weeks after the second vaccination. At the time of blood collection, no active infection was present. This project was submitted for ethical review to and approved by the Bavarian Medical Chamber.

### SARS-CoV-2-derived Peptides

SARS-CoV-2-derived spike overlapping peptide mixes (15-meric peptides with a 4 amino acid overlap each) from the Wuhan wild type (wt) strain were purchased from Miltenyi and JPT and from the alpha (B1.1.7), beta (B1.351), gamma (P.1) and delta (B.1.617.2) mutant strains were purchased from JPT (PepMix SARS.SMUT01, SMUT02, SMUT03-1, SMUT06-1). The alpha mutant (B.1.1.7) contains the following mutations: H0069-, V0070-, Y0144-, N0501Y, A0570D, D0614G, P0681H, T0716I, S0982A, D1118H, with the RBD including a single N501Y mutation. The beta (B.1.351) variant spike protein contains the mutations D0080A, D0215G, L0242-, A0243-, L0244-, K0417N, E0484K, N0501Y, D614G, A0701V, of which K417N, E484K and N501Y are located within the RBD. In contrast, the gamma mutant (P.1) is characterized by mutations: L018F, T020N, P026S, D138Y, R190S, K417T, E484K, N501Y, D614G, H655Y, T1027I, V1176F, and its RBD displays mutations K417T, E484K and N501Y. The delta variant (B.1.617.2) spike protein is characterized by the following mutations compared to wt: T019R, G142D, E156-, F157-, R158G, L452R, T478K, D614G, P681R and D950N, of which L452R and T478K are part of the RBD. Peptide mixes were dissolved in ddH₂O with or without 1% DMSO (Roth).

### Isolation and Freezing of Peripheral Blood Mononuclear Cells and Serum

Blood was collected for peripheral blood mononuclear cell (PBMC) isolation (in S-Monovettes^{®} K3 EDTA (Sarstedt) or equal), as well as for serum isolation (S-Monovettes^{®} Serum (Sarstedt) or equal). The blood was diluted with an equal volume of PBS (Corning) containing 2% fetal bovine serum (FBS; Gibco; heat-inactivated; from Brazil). PBMCs were obtained by density gradient centrifugation using SepMate^{™}-50 tubes (Stemcell Technologies) filled with Lymphoprep^{™} density gradient medium (Stemcell Technologies) according to the manufacturer's instructions. After the isolation, the PBMCs were stained with Acridine Orange and DAPI (solution 18, Chemometec), counted using the NC-250 cell counter (Chemometec), frozen in FBS with 10% DMSO, and stored in liquid nitrogen for at least one week before further experiments. Blood collected for serum isolation was centrifuged 10 min at 2,000xg. Serum was transferred into Eppendorf tubes and frozen at - 20°C until use for further experiments.

### Generation of CHO RBD-His and CHO-NP-Strep Cell Lines

S protein RBD-His consists of the amino acids corresponding to the receptor binding (RBD) domain, which was derived from the S protein nucleotide sequence (positions 22517-23183, amino acids 319 to 541, RVQP....CVNF) of the SARS-CoV-2 Wuhan Hu-1 genome (Genbank accession number MN908947) followed by six histidines. Nucleocapsid protein N-strep consists of the amino acids corresponding to the N protein nucleotide sequence (positions 28290 to 29549) of the SARS-CoV-2 Wuhan Hu-1 genome (Genbank accession number MN908947) followed by a streptavidin tag (NP-Strep). Accordingly, alpha (B.1.1.7) RBD contains a single N501Y mutation, whereas the beta (B.1.351) variant RBD displays K417N, E484K and N501Y and gamma (P.1) RBD mutations K417T, E484K and N501Y. In contrast, the delta variant (B.1.617.2) spike RBD contains L452R, T478K.

The complementary DNA sequences adapted for hamster codon usage were produced synthetically by GeneArt (Life Technologies) by adding the signal sequence METPAQLLFLLLLWLPDTTG (**SEQ ID NO: 8**) at the beginning and cloned into the plasmid vector pcDNA5/FRT via BamHl and Xhol.

The resulting vectors were called pcDNA5/CoV-RBD-His and pcDNA5/CoV-NP-Strep, respectively, and allow for expression and secretion of RBD-His or NP-Strep into the culture medium of mammalian cells under the control of the human cytomegalovirus (CMV) immediate-early enhancer/promoter. The selection for stable clones was done using Hygromycin B after co-transfection with the plasmid pOG44. The vectors were transfected into Flip-lnTM-Chinese hamster ovary (CHO) cells (Life Technologies) using the Lipofectamine 2000 Reagent (Invitrogen, #11668-019), together with the plasmid pOG44, providing site-directed recombination. After selection of a stably expressing clone for each line in Ham's F12 medium supplemented with 10% FBS and 600 µg/ml Hygromycin B, the clones were adapted to ProCHO5 medium (Lonza, #BE12-766Q) supplemented with 4 mM L-glutamine (Biochrom, #K0283).

### Expression and purification of RBD-His and NP-Strep

The various CHO-spike-RBD-His cells and CHO-spike-NP-Strep cells were grown in suspension in ProCHO5, 4 mM L-glutamine and 600 µg/ml Hygromycin B in flasks to submaximal density at 37°C. The supernatants were collected after centrifugation at 400xg for 5 min and subsequent filtration with a 0.22 nm sterile filter (TPP, #99722). The resulting RBD-His or NP-Strep protein-containing medium was immediately frozen and stored at -20°C until protein purification. The cells were continuously grown at 37°C, with splitting and supernatant collection every 3-4 days.

For protein purification, thawed CHO-RBD-His supernatants (0.5 L) were diluted 1:2 in 20 mM sodium phosphate, 0.3 M NaCl, pH 8.0, and loaded onto an equilibrated 1 ml HisTrapTM excel column (GE Healthcare 17-3712-05). After washing the column with wash buffer (20 mM sodium phosphate, 0.3 M NaCl, pH 8.0), RBD-His was eluted with 4 × 1 ml 0.25 M imidazole, 20 mM sodium phosphate, 0.3 M NaCl, pH 8.0. Protein concentration and, hence, content was determined by OD 280 measurement and the relevant fractions were dialyzed (Slyde-A-Lyzer Dialysis Cassette, 10000 MWCO, Thermo Scientific # 66380) against phosphate-buffered saline (PBS from Roth: 137 mM NaCl, 2.7 mM KCI, 10 mM Na2HPO4, 2 mM KH2PO4, pH 7.4, 0.2 µm filtered and steam sterilized) at 4°C for 16 h.

0.5 L CHO-NP-Strep supernatants were diluted 1:2 in 50 mM sodium phosphate, 0.3 M NaCl, pH 8.0, and loaded on an equilibrated 1 ml StrepTrapTM HP column (GE Healthcare 28-9075-46). After washing the column with 50 mM sodium phosphate, 0.3 M NaCl, pH 8.0, NP-Strep was eluted with 4 × 1 ml 20 mM sodium phosphate, 0.3 M NaCl, 2.5 mM desthiobiotin (Sigma, # D 1411) pH 8.0. Protein content was determined by OD 280 measurement and the relevant fractions were dialyzed (Slyde-A-Lyzer Dialysis Cassette, 10000 MWCO, Thermo Scientific # 66380) against PBS at 4°C for 16 h.

For biotinylation of RBD, delta mutantRBD or ACE2, the EZ-Link Micro Sulfo-NHS-LC biotinylation kit (Thermo Scientific # 21935 or #A39257) was used with 20-fold molar excess according the standard procedure instruction, followed by removal of excess biotin by dialysis against 1 I PBS for 16 h at 4°C, using Slide-A-Lyzer^{™} Dialysis Cassettes, 7K MWCO, 0.5 mL (ThermoFisher # 66373).

### Measurement of RBD-His Protein and N-Strep Protein by ELISA

All ELISA measurements were performed at room temperature (RT) and incubations were performed on a microtiter plate shaker. Ni-NTA Hissorb ELISA plates (Qiagen, # 35061) were coated with 100 µl/ well of serially diluted standard protein (5-5000 ng/ml RBD-His, Sino Biological, # 40592-V08B) or diluted test samples (purified RBD-His protein or CHO supernatant) in PBS for 1 h. The coated plates were washed three times with PBST (PBS, 0.1 % Tween-20), blocked with 100 µl/ well blocking solution (PBST, 3% milk powder) for 1 h, and washed again. ELISA plates were incubated with anti-SARS-CoV-2 Spike glycoprotein S1 antibody CR3022 (abcam, # ab273073) diluted 1:2000 in PBST + 1%BSA for 1h. After washing with PBST, the ELISA plates were incubated for 1 h with 100 µl/well of the antihuman IgG detection antibody labelled with POD (Dianova, # 109-035-088, 1:10000 dilution in PBST + 1% BSA). After washing, bound POD was detected by incubation with 100 µl/ well of TMB substrate (Thermo Scientific, #34029) until a maximal optical density (OD) of about 1 to 2 was reached. Finally, the colorimetric reaction was stopped with 100 µl/well stopping solution (1M H2SO4) and the was OD determined at a wavelength of 450 nm with a reference wavelength of 595 nm with in an ELISA plate reader. NP-Strep protein or respective CHO supernatant was analyzed by SARS-CoV-2 Nucleoprotein / NP Elisa Kit (Sino Biological, # KIT40588).

### Qualitative Detection of anti-SARS-CoV2 Antibodies in Human Serum

Anti-SARS-CoV2 antibodies in human serum or in a drop of full blood were detected by a two-step incubation antigen sandwich ELISA using the RBD of the S1 protein of the SARS-CoV2 virus. All procedures were performed at room temperature (RT) and incubations were done on a microtiter plate shaker. ELISA plates were coated with 60 µl/ well RBD-His protein (final concentration 0.45 µg/ml) in coating solution (Candor, #121125) for 1 h. The coated plates were washed three times with PBST (PBS, 0.1 % Tween-20), blocked with 100 µl/well of blocking solution (PBST, 3 % milk powder) for 1 h, and washed again. 30µl serum samples were diluted with 30 µl PBS and transferred to the blocked ELISA plates and incubated for 1h. After washing three times with PBST, the ELISA plates were incubated for 1h with 60 µl/well of biotinylated RBD-His (Bio-RBD-His, final concentration 0.02 µg/ml, diluted in PBST). The plates were washed three times with PBST and incubated with Strep-POD (Jackson Immunoresearch, #016-030-084) diluted in PBST 1:50000 for 1h. After washing five times, bound POD was detected by incubation with 100 µl/ well of TMB substrate (Thermo Scientific, #34029) until a maximal optical density (OD) of about 1 to 2 was reached. Finally, the colorimetric reaction was stopped with 100 µl/ well stopping solution (1M H₂SO₄) and the OD determined at a wavelength of 450 nm with a reference wavelength of 595 nm in a plate reader.

### Statistical Analysis

Statistical analysis was performed using SPSS software version 26. Statistical differences were determined using one-way ANOVA with Tukey's post-hoc test in case data were normally distributed (as assessed by Shapiro-Wilk normality test). Otherwise, data were analyzed using the Kruskal-Wallis nonparametric test.

### Example 1: Neutralization of wt RBD-Biotin binding vs. delta mutant RBD-Biotin binding to immobilized ACE2-His by serum antibodies

All procedures were performed at room temperature (RT) and incubations were done on a microtiter plate shaker. ELISA plates were coated with 60 µl/ well ACE2-His protein with 1 µg/ml in PBS for 1 h. The coated plates were washed three times with PBST (PBS, 0.1 % Tween-20), blocked with 100 µl/well of blocking solution (PBST, 3 % milk powder) for 1 h, and washed again. 60 µl of RBDwt-Bio (0.3 µg/ml) or RBDdelta-Bio (0.3 µg/ml) were co-incubated with various serum sample dilutions (1:2, 1:4, 1:8, 1:16, 1:48, 1:160, 1:1480, 1:1600 in PBST) and transferred to the blocked ELISA plates and incubated for 1h. The plates were washed three times with PBST and incubated with Strep-POD (Jackson Immunoresearch, #016-030-084) diluted in PBST 1:20000 for 1h. After washing four times, bound POD was detected by incubation with 100 µl/ well of TMB substrate (Thermo Scientific, #34029) until a maximal optical density (OD) of about 1 to 2 was reached. Finally, the colorimetric reaction was stopped with 100 µl/ well stopping solution (1M H₂SO₄) and the OD determined at a wavelength of 450 nm with a reference wavelength of 595 nm with in a plate reader.

Then, IC50 values minus blank values were calculated for each sample using results from all dilutions with SigmaPlot v14.5. These IC50 values represent neutralizing serum dilutions. Ratios between IC50s calculated for inhibition of wt vs. delta mutant RBD binding to immobilized ACE2 were used for further analysis.

### Example 2: Neutralization of ACE2-Biotin binding to immobilized wt RBD vs. delta mutant RBD by serum antibodies

SARS-CoV2 mutant spike RBD and nucleocapsid wild type and mutant proteins were expressed in mammalian cells and purified to > 99% purity to be used in the assays. Figures 1A + B show representative examples for the purity after the affinity column and after subsequent dialysis, respectively.

All procedures were performed at room temperature (RT) and incubations were done on a microtiter plate shaker. ELISA plates were coated with 60 µl/ well RBDwt protein or RBDdelta with 2 µg/ml in coating buffer (50mM carbonate buffer, Na₂CO₃/NaHCO₃, pH9.6) for 1 h. The coated plates were washed three times with PBST (PBS, 0.1 % Tween-20), blocked with 100 µl/well of blocking solution (PBST, 3 % milk powder) for 1 h, and washed again. 60µl of ACE2-Bio with 0,3µg/ml was co-incubated with various serum sample dilutions (1:2, 1:4, 1:8, 1:16, 1:48, 1:160, 1:1480, 1:1600 in PBST) and transferred to the blocked ELISA plates and incubated for 1h. The plates were washed three times with PBST and incubated with Strep-POD (Jackson Immunoresearch, #016-030-084) diluted in PBST 1:20000 for 1h. After washing four times, bound POD was detected by incubation with 100 µl/ well of TMB substrate (Thermo Scientific, #34029) until a maximal optical density (OD) of about 1 to 2 was reached. Finally, the colorimetric reaction was stopped with 100 µl/ well stopping solution (1M H₂SO₄) and the OD determined at a wavelength of 450 nm with a reference wavelength of 595 nm with in a plate reader.

Then, IC50 values minus blank values were calculated for each sample using results from all dilutions with SigmaPlot v14.5. These IC50 values represent neutralizing serum dilutions. Ratios between IC50s calculated for inhibition of ACE2 binding to immobilized wt vs. delta mutant RBD were used for further analysis.

All vaccinees showed clear antibody responses against wildtype SARS-CoV2 RBD. This finding occurred regardless of the type of vaccine used - as shown in Figure 2A. We did not detect any anti-nucleocapsid protein antibodies in any vaccinated subjects, except in one subject which had suffered from naturally occurring infection before vaccination.

In comparison, we assessed short-term and long-term immune responses in patients' blood samples up to one year after infection with SARS-CoV2 (Figure 2A). We found specific anti-SARS-CoV2 RBD spike protein and anti-nucleocapsid antibody titers in 90% investigated individuals, as assessed by comparing these titers with non-specific protein binding on the plates. Titers in vaccines were significantly higher than in subjects after natural infection, regardless of the time interval since infection.

Also, inhibition of either wildtype SARS-CoV2 RBD or delta mutant RBD binding to ACE2 was investigated as a correlate for antibody neutralizing potency. We found significantly reduced mean IC50-values for the delta mutant - see Figures 2B-E. Figure 2B shows absolute IC50-values for the inhibition of ACE2 binding to immobilized wt vs. delta mutant RBD, and Figure 2C absolute IC50-values calculated for the inhibition of wt vs. delta mutant RBD binding to immobilized ACE2. The ratios between IC50-values calculated for inhibition of ACE2 binding to immobilized wt vs. delta mutant RBD are shown in Figure 2D. Figure 2E shows ratios between IC50-values calculated for inhibition of wt vs. delta mutant RBD binding to immobilized ACE2. Both, vaccinees and naturally infected subjects showed significantly (p < 0.001 by sign testing) reduced mean IC50-values for the delta mutant, with an average 2,2-fold reduction in vaccinees and an 1,65-fold average reduction in the naturally infected group. Figure 2F shows that results using a drop of finger-stick full blood (about 30 µl) were well comparable to those using sera obtained by venous puncture.

In summary, results from the inhibition assays were comparable, regardless whether RBD or ACE2 was coated onto plates, and whether RBD or ACE was biotinylated and added in solution - and that the assay can be performed with just a drop of finger-stick blood.

### Example 3: T-cell Stimulation and Intracellular Cytokine Staining

*Ex vivo* T-cell responses were analyzed by intracellular cytokine staining (ICS) after stimulation. To this aim, PBMCs were thawed and rested in T cell medium (TCM; RPMI1640 (+GlutaMAX +HEPES, Gibco) + 8% FBS + 1x Penicillin/Streptomycin (Gibco)) containing 1µg/mL DNase (Sigma) for 4-6 hours at 37°C with 5% CO₂ prior to stimulation. Afterwards, 0.5 - 1.5×10⁶ cells per well (96-well plate) were stimulated with 5 µg/mL of the peptide mixes. DdH₂O with or without DMSO (depending on the solvent of the peptides) and PMA (10 µg/mL)/ lonomycin (1 µM) (both from Sigma) were added as negative and positive controls, respectively. The protein transport inhibitor Brefeldin A (10µg/mL, eBioscience) was added simultaneously and cells were incubated for 14-16h at 37°C, 5% CO₂. The next day, cells were washed in PBS + 0.5% BSA (Sigma) and stained extracellularly for 20 min at 4°C (Table 1). Thereafter, cells were fixed with IC Fixation Buffer (Intracellular Fixation & Permeabilization Buffer Set, eBioscience) for 30 min at room temperature. Cells were washed and 1x permeabilization buffer (dilute 10x permeabilization buffer from the intracellular fixation & permeabilization buffer set, eBioscience with 9x ddH20) was added and incubated for 5 min, before intracellular staining for 30 min at room temperature (Table 1). Subsequently, cells were washed twice, resuspended in PBS + 0.5% BSA and analyzed with the MACSQuant10 flow cytometer (Miltenyi).

The data analysis was performed with FCS Express 7 (V. 7.06, De Novo Software). All results were audited, and responses were defined as positive if the percentage of marker-positive cells after peptide stimulation was ≥ two-fold compared to the negative control and >20 marker-positive cells were recorded. The frequency of the negative controls was subtracted from the frequency of marker-positive cells.

### Results

T cell responses were investigated in a first study which showed commonly accepted rates of false negatives and false positives (or inadvertently infected subjects) using a wild type SARS-Cov2 S protein peptide pool derived from the whole amino acid sequence of wildtype protein which was available from the company Miltenyi at the time of the first study (Figure 3). This first study also encompassed vaccinated as well as naturally infected individuals. Similar to results for antibodies, T cell responses to this peptide pool were comparable in all vaccinees regardless of the type of vaccine used. Figure 3 shows the mean activation patterns of tumour necrosis factor (TNF)-alpha (A), interleukin (IL)-2 (B) and of interferon (IFN)-gamma (C) for all subjects. Significant T cell responses were observed both after vaccination and after natural infection.

Follow-up studies then compared peptide pools whose sequences comprised the whole amino acid sequence of wild type and alpha, beta, gamma and delta spike protein mutants, which were obtained from JPT peptides (Figures 4 - 6). Figure 4 shows the mean activation patterns of interferon (IFN)-gamma, Figure 5 of interleukin (IL)-2 and Figure 6 of tumour necrosis factor (TNF)-alpha for all subjects. Specific antibody reactivity and T cell response to SARS-CoV2 mutants alpha, beta, gamma and delta were comparable to wild type in all individuals, with no evidence for reduced responsiveness to any of the mutants (Figures 4 - 6). In comparison, we also tested samples of non infected individuals who were all identified as negative (not shown in the Figure 4 - 6 to enhance legibility). In contrast, significantly reduced T cell responses were observed 1 year after naturally occurring infection compared to vaccines for stimulation: especially, alpha-, beta-, gamma and delta mutant S protein peptide-induced IFN-gamma responses, but also alpha-, beta- and delta mutant-induced IL-2 responses and beta- and delta-induced TNF-alpha responses were significantly lower in subjects which had been infected 1 year before.

We also calculated comparisons of mean T cell responses (TNF alpha and IL-2) with mean anti-RBD and anti-N antibody titers of each group. We observed no relevant correlation between parameters in either vaccinated subjects nor in individuals after naturally occurring infection.

**Table 1:**

| | Marker | Fluorochrome | Clone | Manufacturer | Cat. No. |
|---|---|---|---|---|---|
| Extracellular Staining | Live/dead | fixable Aqua | - | ThermoFisher | L34965 |
| | CD3 | VioBlue | BW264/56 | Miltenyi | 170-081-046 |
| | CD4 | APC | VIT4 | Miltenyi | 130-113-210 |
| | CD8 | PerCP | BW135/80 | Miltenyi | 130-113-160 |
| Intracellular Staining | IFN | PE | 45-15 | Miltenyi | 130-113-493 |
| | CD154 | FITC | 5C8 | Miltenyi | 130-113-606 |
| | TNF | PE-Vio770 | cA2 | Miltenyi | 130-120-492 |
| | IL-2 | APC-Vio 770 | REA689 | Miltenyi | 130-111-492 |

### Nucleotide and amino acid sequences

SEQ ID NO: 1: Amino acid sequence of the wt RBD of the S protein of wild type (wt) SARS-CoV-2 (N501 of the S protein is shown in bold)

MRVQPTESIVRFPNITNLCPFGEVFNATRFASVYAWNRKRISNCVADYSVLYNSASFSTFKC
YGVSPTKLNDLCFTNVYADSFVIRGDEVRQIAPGQTGKIADYNYKLPDDFTGCVIAWNSNNL
DSKVGGNYNYLYRLFRKSNLKPFERDISTEIYQAGSTPCNGVEGFNCYFPLQSYGFQPTNG
VGYQPYRVVVLSFELLHAPATVCGPKKSTNLVKNKCVNF

SEQ ID NO: 2: Amino acid sequence of the RBD of the S protein of alpha SARS-CoV-2 (residue 501 of the S protein mutated to Y in the alpha mutant is shown in bold)

M__R__V__Q__P__T__E__S__I__V__R__F__P__N__I__T__N__L__C__
P__F__G__E__V__F__N__A__T__R__F__A__S__V__Y__A__W__N__R__K__
R__I__S__N__C__V__A__D__Y__S__V__L__Y__N__S__A__S__F__S__T__
F__K__C__Y__G__V__S__P__T__K__L__N__D__L__C__F__T__N__V__Y__
A__D__S__F__V__I__R__G__D__E__V__R__Q__I__A__P__G__Q__T__G__
K__I__A__D__Y__N__Y__K__L__P__D__D__F__T__G__C__V__I__A__W__
N__S__N__N__L__D__S__K__V__G__G__N__Y__N__Y__L__Y__R__L__F__
R__K__S__N__L__K__P__F__E__R__D__I__S__T__E__I__Y__Q__A__G__
S__T__P__C__N__G__V__E__G__F__N__C__Y__F__P__L__Q__S__Y__G__
F__Q__P__T__Y__G__V__G__Y__Q__P__Y__R__V__V__V__L__S__F__E__
L__L__H__A__P__A__T__V__C__G__P__K__K__S__T__N__L__V__K__N__
K__C__V__N__F__*

SEQ ID NO: 3: Amino acid sequence of the RBD of the S protein of beta SARS-CoV-2 (residues 417, 484, and 501 of the S protein mutated this beta mutant are shown in bold)

M__R__V__Q__P__T__E__S__I__V__R__F__P__N__I__T__N__L__C__
P__F__G__E__V__F__N__A__T__R__F__A__S__V__Y__A__W__N__R__K__
R__I__S__N__C__V__A__D__Y__S__V__L__Y__N__S__A__S__F__S__T__
F__K__C__Y__G__V__S__P__T__K__L__N__D__L__C__F__T__N__V__Y__
A__D__S__F__V__I__R__G__D__E__V__R__Q__I__A__P__G__Q__T__G__
N__I__A__D__Y__N__Y__K__L__P__D__D__F__T__G__C__V__I__A__W__
N__S__N__N__L__D__S__K__V__G__G__N__Y__N__Y__L__Y__R__L__F__
R__K__S__N__L__K__P__F__E__R__D__I__S__T__E__I__Y__Q__A__G__
S__T__P__C__N__G__V__K__G__F__N__C__Y__F__P__L__Q__S__Y__G__
F__Q__P__T__Y__G__V__G__Y__Q__P__Y__R__V__V__V__L__S__F__E__
L__L__H__A__P__A__T__V__C__G__P__K__K__S__T__N__L__V__K__N__
K__C__V__N__F__ *

SEQ ID NO: 4: Amino acid sequence of the RBD of the S protein of gamma SARS-CoV-2 (residues 417, 484, and 501 of the S protein mutated in this gamma mutant are shown in bold)

M__R__V__Q__P__T__E__S__I__V__R__F__P__N__I__T__N__L__C__
P__F__G__E__V__F__N__A__T__R__F__A__S__V__Y__A__W__N__R__K__
R__I__S__N__C__V__A__D__Y__S__V__L__Y__N__S__A__S__F__S__T__
F__K__C__Y__G__V__S__P__T__K__L__N__D__L__C__F__T__N__V__Y__
A__D__S__F__V__I__R__G__D__E__V__R__Q__I__A__P__G__Q__T__G__
T__I__A__D__Y__N__Y__K__L__P__D__D__F__T__G__C__V__I__A__W__
N__S__N__N__L__D__S__K__V__G__G__N__Y__N__Y__L__Y__R__L__F__
R__K__S__N__L__K__P__F__E__R__D__I__S__T__E__I__Y__Q__A__G__
S__T__P__C__N__G__V__K__G__F__N__C__Y__F__P__L__Q__S__Y__G__
F__Q__P__T__Y__G__V__G__Y__Q__P__Y__R__V__V__V__L__S__F__E__
L__L__H__A__P__A__T__V__C__G__P__K__K__S__T__N__L__V__K__N__
K__C__V__N__F__ __*

SEQ ID NO: 5: Amino acid sequence of the RBD of the S protein of delta SARS-CoV-2 (residues 452 and 478 of the S protein mutated in this delta mutant are shown in bold)

M__R__V__Q__P__T__E__S__I__V__R__F__P__N__I__T__N__L__C__
P__F__G__E__V__F__N__A__T__R__F__A__S__V__Y__A__W__N__R__K__
R__I__S__N__C__V__A__D__Y__S__V__L__Y__N__S__A__S__F__S__T__

F__K__C__Y__G__V__S__P__T__K__L__N__D__L__C__F__T__N__V__Y__
A__D__S__F__V__I__R__G__D__E__V__R__Q__I__A__P__G__Q__T__G__
K__I__A__D__Y__N__Y__K__L__P__D__D__F__T__G__C__V__I__A__W__
N__S__N__N__L__D__S__K__V__G__G__N__Y__N__Y__R__Y__R__L__F__
R__K__S__N__L__K__P__F__E__R__D__I__S__T__E__I__Y__Q__A__G__
S__K__P__C__N__G__V__E__G__F__N__C__Y__F__P__L__Q__S__Y__G__
F__Q__P__T__N__G__V__G__Y__Q__P__Y__R__V__V__V__L__S__F__E__
L__L__H__A__P__A__T__V__C__G__P__K__K__S__T__N__L__V__K__N__
K__C__V__N__F __*

SEQ ID NO: 6: Amino acid sequence of the wt RBD with C-terminal His-tag of the S protein of wt SARS-CoV-2

MRVQPTESIVRFPNITNLCPFGEVFNATRFASVYAWNRKRISNCVADYSVLYNSASFSTFKC
YGVSPTKLNDLCFTNVYADSFVIRGDEVRQIAPGQTGKIADYNYKLPDDFTGCVIAWNSNNL
DSKVGGNYNYLYRLFRKSNLKPFERDISTEIYQAGSTPCNGVEGFNCYFPLQSYGFQPTNG
VGYQPYRVVVLSFELLHAPATVCGPKKSTNLVKNKCVNFHHHHHH

SEQ ID NO: 7: Amino acid sequence of the wt RBD with N-terminal signal sequence and C-terminal His-tag of the S protein of wt SARS-CoV-2

M__E__T__P__A__Q__L__L__F__L__L__L__L__W__L__P__D__T
T__G__R__V__Q__P__T__E__S__I__V__R__F__P__N__I__T__N__L__C__
P__F__G__E__V__F__N__A__T__R__F__A__S__V__Y__A__W__N__R__K__
R__I__S__N__C__V__A__D__Y__S__V__L__Y__N__S__A__S__F__S__T__
F__K__C__Y__G__V__S__P__T__K__L__N__D__L__C__F__T__N__V__Y__
A__D__S__F__V__I__R__G__D__E__V__R__Q__I__A__P__G__Q__T__G__
K__I__A__D__Y__N__Y__K__L__P__D__D__F__T__G__C__V__I__A__W__
N__S__N__N__L__D__S__K__V__G__G__N__Y__N__Y__L__Y__R__L__F__
R__K__S__N__L__K__P__F__E__R__D__I__S__T__E__I__Y__Q__A__G__
S__T__P__C__N__G__V__E__G__F__N__C__Y__F__P__L__Q__S__Y__G__
F__Q__P__T__Y__G__V__G__Y__Q__P__Y__R__V__V__V__L__S__F__E__
L__L__H__A__P__A__T__V__C__G__P__K__K__S__T__N__L__V__K__N__
K__C__V__N__F__H__H__H__H__H__H__*

SEQ ID NO: 8: signal sequence: METPAQLLFLLLLWLPDTTG

### References

Bilich T, Nelde A, Heitmann JS, Maringer Y, Roerden M, Bauer J, Rieth J, Wacker M, Peter A, Hörber S, Rachfalski D, Märklin M, Stevanovic S, Rammensee HG, Salih HR, Walz JS. T cell and antibody kinetics delineate SARS-CoV-2 peptides mediating long-term immune responses in COVID-19 convalescent individuals. Sci Transl Med 2021;13 (590) eabf7517; doi: 10.1126/scitranslmed.abf7517
Baden LR, El Sahly HM, Essink B, Kotloff K, Frey S, Novak R, Diemert D, Spector SA, Rouphael N, Creech CB, et al. Efficacy and Safety of the mRNA-1273 SARS-CoV-2 Vaccine. N. Engl. J. Med. 2020; 384: 403-416.
Carreño JM, Alshammary H, Singh G, Raskin A, Amanat F, Amoako A, Gonzalez-Reiche AS, van de Guchte A, PARIS study group, Srivastava K, Sordillo EM, Sather DN, van Bakel H, Krammer F, Simon V. Reduced neutralizing activity of post-SARS-1 CoV-2 vaccination serum against variants B.1.617.2, B.1.351, B.1.1.7+E484K and a sub-variant of C.37. medRxiv preprint doi: https://doi.org/10.1101/ 2021.07.21.21260961; posted on July 23, 2021
Choi A, Koch M, Wu K, Dixon G, Oestreicher J, Legault H, Stewart Jones GBE, Colpitts T, Pajon R, Bennett H, Carfi A, Edwards DK. Serum Neutralizing Activity of mRNA-1273 against SARS-CoV-2 Variants. BioRxive preprint posted on June 28, 2021
Gooch KE et al. Heterosubtypic cross-protection correlates with cross-reactive interferon-gamma secreting lymphocytes in the ferret model of influenza. Sci. Rep. 2019; 9, 1-10
Grifoni, A. et al. Targets of T Cell Responses to SARS-CoV-2 Coronavirus in Humans with COVID-19 Disease and Unexposed Individuals. Cell 2020; 181, 1489-1501.e15
Hoffmann M, Kleine-Weber H, Schroeder S, Drosten CS. SARS CoV-2 Cell Entry Depends on ACE2 and TMPRSS2 and Is Blocked by a Clinically Proven Protease Inhibitor. Cell 2020; 181: 271-280e8.
Kim S, Liu Y, Le Zi, Dicker J, Cao Y, Zhang XF, Im W. Differential Interactions Between Human ACE2 and Spike RBD of SARSCoV-2 Variants of Concern. bioRxiv preprint doi: https://doi.org/10.1101/2021.07.23.453598
Krammer F. SARS-CoV-2 vaccines in development. Nature 2020; 586: 516-527
Kreer C, Zehner M, Weber T, Ercanoglu MS, Gieselmann L, Rohde C, Halwe S, Korenkov M, Schommers P, Vanshylla K, V. Di Cristanziano, H. Janicki, R. Brinker, A. Ashurov, V. Krahling, A. Kupke, H. Cohen-Dvashi, M. Koch, J. M. Eckert, S. Lederer, N. Pfeifer, T. Wolf, M. J. G. T. Vehreschild, C. Wendtner, R. Diskin, H. Gruell, S. Becker, F. Klein, Longitudinal Isolation of Potent Near-Germline SARS-CoV-2-Neutralizing Antibodies from COVID-19 Patients. Cell 2020; 182, 843-854.e12. doi:10.1016/j.cell.2020.06.044
Liu J, Liu Y, Xia H, Zou J, Weaver SC, Swanson KA, Cai H, Cutler M, Cooper D, Muik A, Jansen KU, Sahin U, Xie X, Dormitzer PR, Shi PY. BNT162b2-elicited neutralization of B.1.617 and other SARS-CoV-2 variants. Nature. 2021;596(7871):273-275. doi: 10.1038/s41586-021-03693-y.
Long QX, Tang XJ, Shi QL, Li Q, Deng HJ, Yuan J, Lu JL, Zhao R, Li X J, Niu P, Yang B, Wu, H, Wang W, Song H, Huang B, Zhu N, et al. Genomic characterisation and epidemiology of 2019 novel coronavirus: implications for virus origins and receptor binding. Lancet 2020; 395: 565-574
Lumley SF et al. Antibody Status and Incidence of SARS-CoV-2 Infection in Health Care Workers. N Engl J Med 2020; doi:10.1056/nejmoa2034545
Ogbe A, Kronsteiner B, Skelly DT et al. T cell assays differentiate clinical and subclinical SARS-CoV-2 infections from cross-reactive antiviral responses. Nat Commun 2021; 12: 2055. https://doi.org/10.1038/s41467-021-21856-3
Payne et al., Sustained T cell immunity, protection and boosting using extended dosing intervals of BNT162b2 mRNA vaccine. Pre-publication 2021. https://www.pitchstudy.org/PITCH_Dosing_ Interval_23072021.pdf
Planas D, Veyer D, Baidaliuk A, Staropoli I, Guivel-Benhassine F, Rajah MM, Planchais C, Porrot F, Robillard N, Puech J, Prot M, Gallais F, Gantner P, Velay E, Le Guen J, Kassis-Chikhani N, Edriss D, Belec L, Seve A, Courtellemont L, Péré H, Hocqueloux L, Fafi-Kremer S, Prazuck T, Mouquet H, Bruel T, Simon-Loriere E, Rey FA, Schwartz O. Reduced sensitivity of SARS-CoV-2 variant Delta to antibody neutralization. Nature 2021; published on July 8, 2021; https://doi.org/10.1038/s41586-021-03777-9
Polack FP, Thomas SJ, Kitchin N, Absalon J, Gurtman A, Lockhart S, Perez JL, Perez G, Moreira ED, Zerbini C, et al. Safety and Efficacy of the BNT162b2 mRNA Covid-19 Vaccine. N. Engl. J. Med. 2020; 383: 2603-2615.
Rodda LB, Netland J, Shehata L, Pruner KB, Morawski PA, Thouvenel CD, Takehara KK, Eggenberger J, Hemann EA, Waterman HR, Fahning ML, Chen Y, Hale M, Rathe J, Stokes C, Wrenn S, Fiala B, Carter L, Hamerman JA, King NP, Gale Jr. M, Campbell DJ, Rawlings DJ, Pepper M. Functional SARS-CoV-2-Specific Immune Memory Persists after Mild COVID-19. Cell 2021; 184, 169-183.e17. doi:10.1016/j.cell.2020.11.029
Shang J, Ye G, Shi K, Wan Y, Luo C, Aihara H, Geng Q, Auerbach A, Li F. Structural basis of receptor recognition by SARS-CoV-2. Nature 2020; 581: 221-224
Skelly, DT, Harding WAC, Gilbert-Jaramillo WJ, Knight WML, Longet S, Brown A, Adele S, Adland E, Brown H, Stafford L. Vaccine-induced immunity provides more robust heterotypic immunity than natural infection to emerging SARS CoV-2 variants of concern. Research Square 2021; https://doi.org/10.21203/rs.3.rs-226857
Sridhar S et al. Cellular immune correlates of protection against symptomatic pandemic influenza. Nature Med 2013; 19:1305-1312. doi:10.1038/nm.3350
Volz E, Hill V, McCrone JT, Price A, Jorgensen D, O'Toole Á, Southgate J, Johnson R, Jackson B., Nascimento F.F., et al. Evaluating the Effects of SARS-CoV-2 Spike Mutation D614G on Transmissibility and Pathogenicity. Cell 2021; 184: 64-75.e11
Voysey M, Clemens SAC, Madhi SA, Weckx LY, Folegatti PM, Aley PK, Angus B, Baillie VL, Barnabas SL, Bhorat QE, et al. Safety and efficacy of the ChAdOx1 nCoV-19 vaccine (AZD1222) against SARS-CoV-2: an interim analysis of four randomised controlled trials in Brazil, South Africa, and the UK. Lancet. 2020; 397: 99-111.
Walls AC, Park YJ, Tortorici MA, Wall A, McGuire AT, Veesler D. Structure, Function, and Antigenicity of the SARS-CoV-2 Spike Glycoprotein. Cell 2020; 181: 281-982 292.e6
Wang Z, Muecksch Z, Schaefer-Babajew D, Finkin S, Viant C, Gaebler C, Hoffmann HH, Barnes CO, Cipolla M, Ramos V, Oliveira TY, Cho A, Schmidt F, Da Silva J, Bednarski E, Aguado L, Yee J, Daga M, Turroja M, Millard KG, Jankovic M, Gazumyan A, Zhao Z, Rice CM, Bieniasz PD, Caskey M, Hatziioannou T, Nussenzweig MC. Naturally enhanced neutralizing breadth against SARS-CoV-2 one year after infection. Nature 2021; https://doi.org/10.1038/ s41586-021-03696-9
Wilkinson TM et al. Preexisting influenza-specific CD4 + T cells correlate with disease protection against influenza challenge in humans. Nat. Med. 2012; 18, 274-280

## Claims

1. A kit for determining the antibody status, especially of neutralizing antibodies, against SARS-CoV-2 in a subject, comprising:
(i) a solid support having attached an immobilized protein at least on a surface area of said solid support,
(ii) a soluble protein, and
(iii) a detection system for detecting binding of the soluble protein to the immobilized protein;
wherein
(a) said immobilized protein is or comprises ACE2 or a fragment capable of binding to a receptor binding domain (RBD) of an S protein of SARS-CoV-2, and said soluble protein is or comprises an RBD of the S protein of SARS-CoV-2, or
(b) said immobilized protein is or comprises an RBD of an S protein of SARS-CoV-2, and said soluble protein is or comprises ACE2 or a fragment thereof capable of binding to the RBD of the S protein of SARS-CoV-2.

2. The kit according to claim 1, comprising, as said RBD, a first RBD of the S protein of a first SARS-CoV-2 strain and a second RBD of the S protein of a second SARS-CoV-2 strain, wherein said second RBD is a mutated variant of said first RBD, for determining the immunization or antibody status of the subject against the second SARS-CoV-2 strain compared to the immunization or antibody status of the subject against the first SARS-CoV-2 strain.

3. The kit according to claim 1 or 2, wherein said solid support has attached:
a first RBD of the S protein of a first SARS-CoV-2 strain on a first surface area of said support, and
a second RBD of the S protein of a second SARS-CoV-2 strain on a second, separate, surface area of said support, and
optionally RBDs of further SARS-CoV-2 strains on further separate surface areas of said support.

4. The kit according to any one of claims 1 to 3, wherein said solid support is a microarray having attached two or more spots of said immobilized protein.

5. The kit according to claim 3, wherein said solid support is a microarray having attached one or more spots of said first RBD and one or more spots of said second RBD, and optionally one or more further spots of further variants of said RBD.

6. The kit according to any one of claims 1 to 5, wherein said RBD is produced by a process comprising cultivating a mammalian cell containing a nucleic acid molecule comprising a polynucleotide encoding said RBD and expressing said RBD in said cell.

7. A method for determining the antibody status, especially of neutralizing antibodies, against SARS-CoV-2 in a subject, preferably in a subject after vaccination against Covid-19, said method comprising:
(A) providing a solid support having attached an immobilized protein,
(B) mixing a blood or serum sample derived from said subject with a soluble protein,
(C) contacting the mixture obtained in step (B) with a surface area of said solid support, said surface area having attached said immobilized protein, and
(D) detecting binding of said soluble protein to said immobilized protein using a detection system;
wherein
(a) said immobilized protein is or comprises ACE2 or a fragment capable of binding to a receptor binding domain (RBD) of an S protein of SARS-CoV-2, and said soluble protein is or comprises an RBD of the S protein of SARS-CoV-2, or
(b) said immobilized protein is or comprises an RBD of an S protein of SARS-CoV-2, and said soluble protein is or comprises ACE2 or a fragment thereof capable of binding to the RBD of the S protein of SARS-CoV-2.

8. The method of claim 7, wherein:
step (B) comprises preparing two or more different dilutions of said blood or serum sample, and mixing each of said two or more different dilutions with said soluble protein,
step (C) comprises contacting each of the two or more mixtures obtained in step (B) with a separate surface area of said solid support, each surface area having attached said immobilized protein, and
step (D) comprises detecting binding of said soluble protein to said immobilized protein using a detection system on each surface area that was contacted with a mixture obtained in step (B).

9. The method of claim 7 or 8, wherein said soluble protein is biotinylated and the detection system comprises a streptavidin-labeled detection protein, such as a streptavidin-labeled peroxidase.

10. The method according to any one of claims 7 to 9, wherein said subject is vaccinated against Covid-19, has recovered from Covid-19, or was previously infected by SARS-CoV-2.

11. A method of determining the T-cell response to SARS-CoV-2 in a subject, comprising:
(i) obtaining a blood sample from the subject,
(ii) isolating peripheral blood mononuclear cells from said blood sample,
(iii) incubating said cells obtained in step (ii) with a SARS-CoV-2 S protein, an RBD fragment of an S protein, or one or more peptides from an S protein or an RBD of SARS-CoV-2,
(iv) staining extracellular T-cell markers selected from the group consisting of CD3, CD4, and CD8 of the cells obtained in step (iii),
(v) fixing the cells obtained in step (iv) on a solid support,
(vi) staining intracellular T-cell markers selected from the group consisting of IFN, TNF, and IL-2, followed by resuspending the cells, and
(vii) analyzing extracellular and intracellular T-cell markers of resuspended cells by flow cytometry using signals due to the stained markers.

12. The method according to claim 11, wherein at least the extracellular marker CD4 and one of said intracellular markers are analyzed in step (vii); or wherein at least the extracellular markers CD3, CD4, and CD8 and one of said intracellular markers are analyzed in step (vii).

13. The method according to claim 11 or 12, wherein said SARS-CoV-2 S protein, said RBD fragment of an S protein, or said one or more peptides from an S protein or an RBD are from the wild-type SARS-CoV-2 strain or from a mutant SARS-CoV-2 strain, such as the alpha, beta, gamma, or delta SARS-CoV-2 strain.

14. The method according to any one of claims 11 to 13, further comprising step (viii):
comparing fluorescence intensities, analyzed in step (vii), of stained T-cell markers or the cell population stained for at least one extracellular and at least one intracellular T-cell marker of said subject with a control; or
comparing fluorescence intensities, analyzed in step (vii), of stained T-cell markers or the cell population stained for at least one extracellular and at least one intracellular T-cell marker of said subject with the respective intensities or cell population of a vaccinated control subject or an average of recently vaccinated control subjects.

15. The method according to claim 11 to 14, comprising, in step (iii),
incubating said cells with a SARS-CoV-2 S protein, an RBD fragment of an S protein, or one or more peptides from an S protein or an RBD of the wild-type SARS-CoV-2 strain in a first container and,
incubating said cells with a SARS-CoV-2 S protein, an RBD fragment of an S protein, or one or more peptides from an S protein or an RBD of a mutant SARS-CoV-2 strain in a second container, and
comprising in step (viii) comparing stained T-cell marker(s) in said cells incubated in said first container with that/those incubated in said second container.
